Europäisches Patentamt

European Patent Office

Office européen des brevets

(19)

(11) **EP 0 753 305 B1**

(12) **EUROPEAN PATENT SPECIFICATION**

(45) Date of publication and mention
of the grant of the patent:
**30.08.2000   Bulletin 2000/35**

(51) Int Cl.[7]: **A61K 35/78**

(21) Application number: **96106962.2**

(22) Date of filing: **03.05.1996**

(54) **Use of crude plant extract for the preparation of a medicament for treating HIV infection**

Verwendung eines Rohpflanzenextraktes zur Herstellung eines Arzneimittels zur Behandlung von HIV-Infektionen

Utilisation d' un extrait brut des plantes pour la préparation des médicaments pour le traitement d'infections à VIH

(84) Designated Contracting States:
**CH DE FR GB LI SE**

(30) Priority: **13.07.1995 JP 20053295**
**09.02.1996 JP 4833796**

(43) Date of publication of application:
**15.01.1997   Bulletin 1997/03**

(73) Proprietor: **Showa Shell Sekiyu Kabushiki Kaisha Tokyo (JP)**

(72) Inventors:
• **Hozumi, Toyoharu, c/o Showa Shell Sekiyu K.K. Tokyo (JP)**
• **Ooyama, Haruo, c/o Showa Shell Sekiyu K.K. Tokyo (JP)**
• **Shiraki, Kimiyasu Toyama-shi, Toyama (JP)**
• **Kurokawa, Masahiko Kosugi-machi Imizu-gun Toyama (JP)**
• **Kageyama, Seiji Toyoma-shi Toyama (JP)**
• **Sato, Hitoshi Toyama-shi Toyama (JP)**
• **Namba, Tsuneo Toyama-shi Toyama (JP)**
• **Tsuchie, Hideaki Yao-shi Osaka (JP)**
• **Kurimura, Takashi Suita-shi Osaka (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 568 001          WO-A-94/18993**

• **CHEMICAL AND PHARMACEUTICAL BULLETIN, vol. 43, no. 4, April 1995, JAPAN, pages 641-648, XP000196426 S. EL-MEKKAWY ET AL: "Inhibitory Effects of Egyptian Folk Medicines on Human Immunodeficiency Virus (HIV) Reverse Transcriptase"**
• **PLANTA MEDICA, vol. 42, 1981, GERMANY, pages 69-74, XP000196427 M. TAKECHI ET AL: "Purification and Characterization of Antiviral Substance from the Bud of Syzygium aromatica"**
• **PATENT ABSTRACTS OF JAPAN vol. 18, no. 262 (C-1201), 19 May 1994 & JP-A-06 040930 (SUMITOMO METAL IND LTD), 15 February 1994,**

**Description**

This invention relates to the use of a crude extract for preparing a medicament for treating HIV infection.

HIV (human immunodeficiency virus) is the pathogen of acquired immunodeficiency syndrome (AIDS) whose disease concept was established in the 1980's. Soon after HIV infection, the antibody reaction changes to positive via influenza-like initial symptoms (3 to 15 days). After a latent period of one to several years, T-cell insufficiency is developed which subsequently causes AIDS-related syndrome (lymph node enlargement, body weight loss, pyrexia, sweating and chronic diarrhea), AIDS (worsening of the above symptoms, pneumocystis carinii pneumonia, opportunistic infection of candidasis and Kaposi sarcoma) and sometimes neurosis.

Only AZT and ddI, which belong to the dideoxynucleotide family, are now used in the clinical field for the treatment of AIDS other than the HIV symptomatic therapy. In many cases, treatment with these drugs is forced to be suspended due to their side effects. In addition to this, the generation of resistant viruses is causing a serious problem. Since these problems mean that a prolonged period of continuous administration cannot be made by a single drug administration, development of a variety of drugs is a pressing need.

Starting around 1989, development of HIV protease inhibitors has been reported in succession as new generation drugs instead of dideoxynucleotides. It has been confirmed that these HIV protease inhibitors are effective on various viral strains including HIV-2 type virus and show sufficient *in vitro* activities. However, these HIV protease inhibitors have a disadvantage in that their activities are reduced due to their nonspecific reaction with blood protein.

On the other hand, development of anti-HIV agents making use of crude drugs is also in progress (a crude drug is a drug which is obtained from a natural source such as a plant). It is said that licopyranocoumarin extracted from Glycyrrhiza Root shows anti-HIV activity at a concentration of 20 μg/ml (Miyamoto *et al.,* 1st Meeting Jap.. Soc. AIDS Res. Abstract, 1987).

Also, it has been reported that water or alcohol extracts of 6 Indonesian crude drugs Supratul (*Sindora sumatrana*)*,* Klules (*Helicteres isora*)*,* Mahoni (*Swietenia mahagoni*), Sambiloto (*Andrographis paniculate*), Temu ireng (*Curcuma aeruginosa*) and Benaluteh (*Loranthus parasiticus*) (scientific names are shown in parentheses) show anti-HIV activity at respective concentrations of 134 μg/ml (water extract), 65 μg/ml (methanol extract), 28.4 μg/ml (methanol extract), 170 μg/ml (water extract), 323 μg/ml (water extract) and 79 μg/ml (water extract) (JP-A-6-40930, the term "JP-A" as used herein means an "unexamined published Japanese patent application").

In Chem. Pharm. Bull. 43(4) 641-648 (1995) it is reported that extracts inter alia of fruits of Terminalia chebula showed a significant inhibitory activity on human immuno-deficiency virus-1 reverse transcriptase. It is an object of the present invention to provide a number of agents which are effective in treating the onset of HIV and have fewer side effects, in order to resolve the prior art problems that occur when a single administration of a dideoxynucleotide such as AZT or is carried out.

The inventors of the present invention have examined a broad range of crude drugs conventionally used as therapeutic drugs whose dose, administration route, and the like are well known and which show no, or slight, side effects.

The present invention relates to the use of a one crude extract selected from the group consisting of *Alpinia officinarum* Hance, *Geum japonicum* Thunb., *Paeonia suffruticosa* Andrews, *Phellodendron amurence* Ruprecht, *Punica granatum L.,* Rhus *javanica L., Syzygium aromaticum (L.)* Merr. et Perry and *Terminalia arjuna* Wight et Arn., for the preparation of a medicament for the prevention and treatment of HIV.

A pharmaceutically acceptable carrier used in the medicament can be, e.g., water.

The therapeutically effective amount varies depending on the crude drug being used and is determined based on the crude drug being used.

For example, water is added to 2 to 10 g of at least one crude drug selected from the crude extracts in an amount of 50 ml per g of the crude extract. Then, the water containing the crude extract is boiled down to about a half of the original volume at a heating power to such an extent that the water does not boil over. The thus obtained extract may be orally administered three times a day at a dose of one-third of the extract.

The methods for using the extracts are not particularly limited to the above method. The extract may be concentrated and dried to use as powders. Furthermore, the dried extract obtained may be formulated into any dose form such as granules, tablets, and powders. In these formulations, excipients which are usually pharmaceutically used, such as starch, lactose, and magnesium stearate, may be mixed with the extract.

An example of the present invention is given below by way of illustration. Unless otherwise indicated, all parts percents, ratios, and the like are by weight.

EXAMPLE

Crude extracts from the following parts are used in the example.

1. *Alpinia officinarum* Hance (rhizome)
2. *Geum japonicum* Thunb. (whole plant)
3. *Paeonia suffruticosa* Andrews (root bark)
4. *Phellodendron amurence* Ruprecht (bark)
5. *Punica granatum* L. (root bark and fruit peel)
6. *Rhus javanica* L. (insect gall)
7. *Syzygium aromaticum* (L.) Merr. et Perry (flower bud)
8. *Terminalia arjuna* Wight et Arn. (bark)

A 100 mg portion of powder obtained by pulverizing each of the above crude extracts was mixed with 5 ml of high purity water, the resulting suspension was boiled for 5 minutes and centrifuged for 10 minutes at every 400 g and the thus obtained supernatant fluid was adjusted to 20 mg/ml to be used as an extract in the following experiments.

Next, the experimental method and judging method used in the following anti-HIV activity test are described. The test was carried out in accordance with the method developed by Mitsuya *et al.* (*Proc. Natl. Acad. Sci. USA,* 82: 7096 - 7100, 1985). MT-4 cells established from an HIV-infected patient were infected with HIV-I$_{LAI}$ for 1 hour at an amount of 3,000 TCID$_{50}$/5$\times$10$^5$ cells and mixed with a predetermined concentration of each drug, and a 1 ml portion of the resulting cell suspension was cultured for 4 days after the infection using a 24 well plate. Thereafter, viable cells and dead cells were distinguished by trypan blue staining to count the number of cells which were saved by the influence of the drug from cell death caused by the viral infection. In order to examine the cytotoxicity of the drug, the same test was carried out using a group which was not infected with the HIV, and the results were used in the evaluation of the anti-HIV activity.

In this connection, when the anti-viral activity is examined a plurality of times using the same concentration of extract solution, the test results sometimes differ from one another slightly, which, however, seems to be a judging error formed by a slight difference in the sensitivity due to culture conditions (serum lot, culture plate, cell density and the like).

The drug effect of each crude extract was evaluated in the following manner.

First, the number of viable mock-infected cells to which the crude extract was not added and the number of viable infected cells to which the crude drug extract was not added were counted to estimate the number of survived cells. Next, the number of viable infected cells to which the crude extract was added was counted to estimate the number of survived cells in the presence of the crude drug extract.

Evaluation of each crude extract was made based on the increasing ratio of viable count effected by the administration of the crude extract (viable cell percentage).

The results are shown in Table 1.

In the table, a higher viable cell percentage means a stronger anti-HIV effect. Also, a lower cytotoxicity percentage means a weaker toxicity for cells.

In this test, the anti-HIV effect was judged positive when a viable cell percentage of 10% or more was obtained by the administration of each crude extract.

Each of the crude extracts showed an anti-HIV effect at a concentration of 2 μg/ml or less in the case of *Alpinia officinarum, Phellodendron amurence, Punica granatum, Rhus javanica, Syzygium aromaticum* and *Geum japonicum* and at a concentration of 20 μg/ml or less in the case of *Paeonia suffruticosa* and *Terminalia arjuna.* None of them showed strong cytotoxicity at such concentrations.

Furthermore, in Table 1, some crude drugs showed a lower viable cell percentage at a higher concentration. These results were because many cultured cells were died by the cytotoxicity which was originally possessed by the crude extracts and as a result, the number of viable cells was reduced. However, all tested crude extracts showed anti-HIV activity at a concentration which was lower than that showing the cytotoxicity.

Table 1

| Results of anti-HIV activity test | | | |
|---|---|---|---|
| Extracts | Conc. | Viable count ($\times 10^5$)[1] (cell percentage [1] or cytotoxicity percentage [2]) | |
| | | HIV infection | HIV mock infection (no infection) |
| none | - | 1.8 | 8.4 |
| *Alpinia officinarum* | 2 µg/ml | 3.3 (23) [1] | 8.4 (0) [2] |
| | 20 µg/ml | 3.3 (23) | 7.9 (6) |
| | 200 µg/ml | 4.0 (33) | 5.8 (31) |
| *Paeonia suffruticosa* | 2 µg/ml | 0.9 (0) | 8.1 (4) |
| | 20 µg/ml | 2.7 (14) | 8.8 (0) |
| | 200 µg/ml | 3.7 (29) | 4.9 (42) |
| *Phellodendron amurence* | 2 µg/ml | 3.8 (30) | 6.5 (23) |
| | 20 µg/ml | 2.1 (5) | 2.3 (73) |
| | 200 µg/ml | 0 (0) | 0.2 (98) |
| Punica granatum | 2 µg/ml | 2.8 (15) | 9.0 (0) |
| | 20 µg/ml | 3.8 (30) | 3.5 (58) |
| | 200 µg/ml | 0 (0) | 0 (100) |
| *Terminalia arjuna* | 2 µg/ml | 2.4 (9) | 8.5 (0) |
| | 20 µg/ml | 4.8 (45) | 7.7 (8) |
| | 200 µg/ml | 0 (0) | 0.1 (99) |
| *Rhus javanica* | 2 µg/ml | 2.7 (14) | 5.9 (30) |
| | 20 µg/ml | 1.3 (0) | 1.9 (77) |
| | 200 µg/ml | 0 (0) | 0 (100) |
| *Syzygium aromaticum* | 2 µg/ml | 2.9 (17) | 8.5 (0) |
| | 20 µg/ml | 4.2 (36) | 5.9 (30) |
| | 200 µg/ml | 0 (0) | 0 (100) |
| *Geum japonicum* | 2 µg/ml | 3.1 (20) | 8.2 (2) |
| | 20 µg/ml | 3.7 (29) | 6.7 (20) |
| | 200 µg/ml | 0.6 (0) | 0.6 (93) |
| Dideoxyinosine | 20 µM | 8.6 (103) | 8.7 (0) |
| (ddI) | 50 µM | 8.8 (106) | 8.4 (0) |

[1] Viable cell percentage = $\frac{(A) - (B)}{(C) - (B)} \times 100$

[2] Cytotoxicity percentage = $\frac{(C) - (D)}{(C)} \times 100$

(A): Counts of infected viable cells in the presence of extract
(B): Counts of infected viable cells in the absence of extract
(C): Counts of mock-infected viable cells in the absence of extract
(D): Counts of mock-infected viable cells in the presence of extract

In the above test, an examination was made to find the degree of the effect of the crude extracts in decreasing the number of dead cells caused by HIV. As the action points which generate the anti-HIV activity, inhibition of adsorption of HIV to cells, inhibition of an HIV-originated reverse transcriptase, inhibition of an HIV-originated protease and the like can be exemplified. In the following test, inhibition of HIV-originated reverse transcriptase was examined.

The reverse transcriptase inhibition activity test was carried out in the following manner.

The reverse transcriptase inhibition activity was examined using 7 crude extracts which showed high anti-HIV activity. The reverse transcriptase inhibition activity was measured in accordance with the method of B.J. Poiesz et *al.* (*Proc. Natl. Acad. Sci. USA,* vol.77, no.12, pp.7415 - 7419, 1980).

To a reverse transcriptase reaction solution [30 U/ml AMV reverse transcriptase (manufactured by Pharmacia), 40 mM Tris-HCl (pH 7.8), 4 mM dithiothreitol, 45 mM KCl, 10 mM $MgCl_2$, 50 µg/ml poly(A) $dT_{12-18}$ template primer and 50 µg/ml poly(C) $dG_{12-18}$ template primer] were added each crude drug extract (final concentration, 2, 20 or 200 µg/ml) and labeled deoxytriphosphate (final concentration, 15 µM), followed by 30 minutes of reaction at 37°C. Total volume of the reaction solution was adjusted to 100 μl, and the reverse transcriptase inhibition activity was measured based on the incorporated amount of the label. AZT and ddI do not show reverse transcriptase inhibition activity since these are not phosphorylated by this test. Therefore, AZT and ddI were not examined at this test.

The results are shown in Table 2.

Table 2

| Reverse transcriptase inhibition activity of crude extract | | | |
|---|---|---|---|
| | Reverse transcriptase inhibition activity (%) | | |
| Extracts | Extract conc. (μg/ml) 2 | 20 | 200 |
| *Alpinia officinarum* | - | 54 | 92 |
| *Geum japonicum* | 8 | 29 | 55 |
| *Paeonia suffruticosa* | - | 23 | 55 |
| *Punica granatum* | 17 | 38 | 61 |
| *Terminalia arjuna* | - | 6 | 3 |
| *Syzygium aromaticum* | 11 | 43 | 76 |

Each of the crude extracts excluding *Terminalia arjuna* showed reverse transcriptase inhibition activity in a concentration-dependent fashion, indicating that the reverse transcriptase inhibition action is included in the action mechanism of these crude extracts. On the other hand, the *Terminalia arjuna* extract did not show reverse transcriptase inhibition activity, indicating that it has an action mechanism which is different from that of the prior art dideoxynucleotide extracts.

Thus, it is apparent that there has been provided an anti-HIV composition by the use of the specified crude extracts which hardly shows side effects.

## Claims

1. The use of a crude extract selected from the group consisting of *Alpinia officinarum* Hance, *Geum japonicum* Thunb., *Paeonia suffruticosa* Andrews, *Phellodendron amurence* Ruprecht, *Punica granatum L., Rhus javanica L., Syzygium aromaticum (L.)* Merr. et Perry, and *Terminalia arjuna* Wight et Arn., for the preparation of a medicament for the prevention and treatment of HIV.

2. The use according to claim 1,
   wherein the crude extract is selected from the group consisting of *Phellodendron amurence* Ruprecht, *Rhus javanica L., Punica granatum* L., *Geum japonicum* Thunb., *Syzygium aromaticum* (L.) Merr. et Perry, *Paeonia suffruticosa* Andrews and *Alpinia officinarum* Hance.

3. The use according to claim 1,
   wherein the crude extract comprises Terminalia *arjuna* Wight et Arn.

4. The use according to claim 1,
   wherein the crude extract is selected from the group consisting of *Phellodendron amurence* Ruprecht, *Rhus javanica* L., *Punica granatum* L., *Geum japonicum Thunb.*, *Syzygium aromaticum* (L.) Merr. et Perry, and *Alpinia officinarum* Hance and is used in a concentration of at most 2 μg/ml.

5. The use according to claim 1,
   wherein the crude extract is selected from the group consisting of *Terminalia arjuna* Wight et Arn. and *Paeonia suffruticosa* Andrews and is used in a concentration of at most 20 μg/ml.

## Patentansprüche

1. Verwendung eines Rohextrakts, ausgewählt aus der Gruppe, bestehend aus Alpinia officinarum Hance, Geum japonicum Thunb., Paeonia suffruticosa Andrews, Phellodendron amurence Ruprecht, Punica granatum L., Rhus javanica L., Syzygium aromaticum (L.) Merr. et Perry und aus Terminalia arjuna Wight et Arn., zur Herstellung eines Medikaments zur Vorbeugung und Behandlung von HIV.

2. Verwendung gemäß Anspruch 1, worin der Rohextrakt aus der Gruppe ausgewählt ist, bestehend aus Phellodendron amurence Ruprecht, Rhus javanica L., Punica granatum L., Geum japonicum Thunb., Syzygium aromaticum (L.) Merr. et Perry, Paeonia suffruticosa Andrews und aus Alpinia officinarum Hance.

3. Verwendung gemäß Anspruch 1, worin der Rohextrakt Terminalia arjuna Wight et Arn. umfasst.

4. Verwendung gemäß Anspruch 1, worin der Rohextrakt aus der Gruppe, bestehend aus Phellodendron amurence Ruprecht, Rhus javanica L., Punica granatum L., Geum japonicum Thunb., Syzygium aromaticum (L.) Merr. et Perry, und aus Alpinia officinarum Hance, ausgewählt ist und in einer Konzentration von höchstes 2 µg/ml angewandt wird.

5. Verwendung gemäß Anspruch 1, worin der Rohextrakt aus der Gruppe, bestehend aus Terminalia arjuna Wight et Arn. und Paeonia suffruticosa Andrews, ausgewählt ist und in einer Konzentration von höchstens 20 µg/ml angewandt wird.

**Revendications**

1. Utilisation d'un extrait brut choisi dans le groupe constitué par *Alpinia officinarum* Hance, *Geum japonicum* Thunb., *Paeonia suffruticosa* Andrews, *Phellodendron amurence* Ruprecht, *Punica granatum L., Rhus javanica L., Syzygium aromaticum* (L.) Merr. et Perry et *Terminalia arjuna* Wight et Arn., pour la préparation d'un médicament pour la prévention et le traitement du VIH.

2. Utilisation selon la revendication 1, dans laquelle l'extrait brut est choisi dans le groupe constitué par *Phellodendron amurence* Ruprecht, *Rhus javanica L., Punica granatum L., Geum japonicum* Thunb., *Syzygium aromaticum* (L.) Merr. et Perry, *Paeonia suffruticosa* Andrews et *Alpinia officinarum* Hance.

3. Utilisation selon la revendication 1, dans laquelle l'extrait brut comprend *Terminalia arjuna* Wight et Arn.

4. Utilisation selon la revendication 1, dans laquelle l'extrait brut est choisi dans le groupe constitué par *Phellodendron amurence* Ruprecht, *Rhus javanica L., Punica granatum L., Geum japonicum* Thunb., *Syzygium aromaticum* (L.) Merr. et Perry et *Alpinia officinarum* Hance et est utilisé en une concentration d'au plus 2 µg/ml.

5. Utilisation selon la revendication 1, dans laquelle l'extrait brut est choisi dans le groupe constitué par *Terminalia arjuna* Wight et Arn. et *Paeonia suffruticosa* Andrews et est utilisé en une concentration d'au plus 20 µg/ml.